# EUROPEAN PATENT SPECIFICATION

(11) **EP 3 527 134 B1**
(45) Date of publication and mention of the grant of the patent: **02.12.2026**
(21) Application number: 19167435.7
(22) Date of filing: 19.04.2012
(51) Int. Cl.: A61B 5/145, A61B 5/00, A61M 1/14, A61M 1/16, A61M 1/34

(54) **ELECTROLYTE AND PH MONITORING FOR FLUID REMOVAL PROCESSES**
ELEKTROLYT- UND PH-ÜBERWACHUNG FÜR FLÜSSIGKEITSENTFERNUNGSVERFAHREN
SURVEILLANCE D'ÉLECTROLYTE ET DE PH POUR DES PROCÉDÉS D'ÉLIMINATION DE FLUIDE

(30) Priority: 29.04.2011 US 201161480539 P; 29.04.2011 US 201161480544 P; 29.04.2011 US 201161480541 P; 29.04.2011 US 201161480535 P; 29.04.2011 US 201161480532 P; 29.04.2011 US 201161480530 P; 29.04.2011 US 201161480528 P; 20.03.2012 US 201213424479
(43) Date of publication of application: 21.08.2019
(62) Document number(s) of the earlier application(s) in accordance with Art. 76 EPC: 12722951.6 / 2 701 596
(73) Proprietor: Mozarc Medical US LLC, Minneapolis, MN 55431 (US)
(72) Inventor: GERBER, Martin T., Maple Grove, Minnesota 55369 (US); LYU, Suping, Maple Grove, Minnesota 55311 (US); BURNES, John E., Blaine, MN 55449 (US); VENKATESH, Manda R., Stillwater, Minnesota 55082 (US); PUDIL, Bryant J., Plymouth, Minnesota 55447 (US)
(74) Representative: Greaves Brewster LLP

(56) References cited:
- EP-A2- 0 272 414
- WO-A1-2009/026603
- US-A1- 2005 236 330
- US-A1- 2007 066 928
- US-A1- 2011 077 574

## Description

### RELATED APPLICATION

This application claims priority to U.S. Utility Application No. 13/424,479 filed March 20, 2012, which claims priority U.S. Provisional Application No. 61/480,539, U.S. Provisional Application No. 61/480,544, U.S. Provisional Application No. 61/480,541, U.S. Provisional Application No. 61/480,535, U.S. Provisional Application No. 61/480,532, U.S. Provisional Application No. 61/480,530, and U.S. Provisional Application No. 61/480,528, wherein each priority Provisional Application was filed April 29, 2011.

### FIELD

The present invention relates generally to systems for monitoring indicators of electrolytes or pH in patients for which blood cleaning or fluid removal is indicated, such as patients suffering from kidney disease or heart failure.

### BACKGROUND

Patients who undergo hemodialysis or other procedures that remove solutes and fluid from the blood often die of cardiac complications. Many factors may contribute to such death, including stress placed on the heart due to the increased blood fluid volume in these patients. Increased fluid concentrations and inability to remove waste products from the blood, in some cases, can also contribute to electrolyte and pH imbalance that can affect cardiac contractility and efficiency. Further, rapid changes in fluid volume or pH or electrolyte concentration of the blood during hemodialysis or other fluid removal processes may place additional stress on the heart and may contribute to the high rate of morbidity for patients who undergo blood fluid removal procedures.

When a patient reaches a point where routine blood fluid removal procedures are prescribed, the patient undergoes periodic examinations that allow a healthcare provider to set various parameters of the blood fluid removal procedures, such as the profile of fluid removal, the composition of dialysate or replacement fluid employed, and the like. These examinations typically occur once a month in accordance with current standards of care.

Hemodialysis or similar procedures may occur three to four times a week. Thus, the patient may undergo 10 to 15 or more blood fluid removal sessions before the prescription or parameters are changed. It is possible, for example, that a prescription with regard to a dialysate electrolyte and pH buffer composition will not be appropriate for a patient several days or weeks after the prescription is set. Accordingly, it may be desirable to more frequently determine whether the electrolyte or pH concentration of a fluid used in blood fluid removal sessions is appropriate. In addition, it may be desirable to adjust the concentration or composition of the fluid during a blood fluid removal session in a manner that may improve patient health and reduce morbidity.
US 2004/019312, US 2005/0236330, and EP 0330892 teach blood dialysis apparatus.

### SUMMARY

This disclosure, among other things, describes devices, systems and uses for monitoring indicators of pH or electrolytes in patients for which blood fluid removal sessions are indicated. The monitoring may occur during a blood fluid removal session, and the concentration or composition of buffer or electrolytes may be adjusted based on monitored data acquired during the blood fluid removal session. By monitoring pH or electrolytes, the dialysate of replacement fluid may be adjusted during a session to enhance patient safety. The invention is a system as defined in claim 1.

Also described is a use including initiating a blood fluid removal procedure for a patient in need thereof. The procedure includes use of a fluid selected from a dialysate fluid or a replacement fluid. The fluid has an initial pH buffer composition or electrolyte composition. The use further includes monitoring an indicator of blood electrolyte concentration or blood pH of the patient during the blood fluid removal session, and adjusting the pH buffer composition or the electrolyte composition of the fluid based on a value of the monitored indicator. The monitoring may be of blood before or after the blood has passed through the blood fluid removal device, or may be of fluid removed from the blood. In some embodiments, data acquired from monitoring performed on blood before and after passing through the blood fluid removal device is compared to data acquired from fluid (e.g., dialysate) before and after passing through blood fluid removal media of the device, and based on the comparison, the pH buffer composition or the electrolyte composition of the fluid is adjusted.

One or more embodiments of the systems, devices and uses described herein may provide one or more advantages over prior systems, devices and uses for blood fluid removal in patients. Such advantages will be apparent to those skilled in the art upon reading the following detailed description.

### BRIEF DESCRIPTION OF THE DRAWINGS

The accompanying drawings, which are incorporated into and form a part of the specification, illustrate several embodiments of the present disclosure and, together with the description, serve to explain the principles of the disclosure. The drawings are only for the purpose of illustrating embodiments of the disclosure and are not to be construed as limiting the disclosure.
**FIGS. 1-3** are schematic block diagrams showing interaction of blood fluid removal devices with a patient showing flow of blood (dashed arrows) and fluid (solid arrows), which blood fluid removal devices may be used in various embodiments not forming part of the claimed invention.
**FIG. 4** is a schematic block diagram showing some selected components of an embodiment of a sensor device.
**FIGS. 5-6** are schematic block diagrams showing sensors and blood flow between patients and a blood fluid removal devices. Fig 5. is a schematic block on an embodiment not forming part of the claimed invention. Fig 6 is a schematic block on an embodiment of the claimed invention.
**FIGS. 7-8** are schematic block diagrams showing flow paths and some control mechanisms (closed loop: **FIG. 7****;** open loop: **FIG. 8**) for controlling flow of concentrate into fluid for use in a blood fluid removal process based on monitored pH or electrolytes.
**FIGS. 9-10** are schematic block diagrams of some components of blood fluid removal devices that are configured to adjust pH or electrolyte concentrations of fluids in response to data regarding monitored pH or electrolyte levels in blood. Figs 9 and 10 are embodiments not forming part of the claimed invention.
**FIGS. 11-13** are flow diagrams illustrating overviews of general uses in accordance with embodiments described herein. Figs 11 and 13 are flow diagrams not forming part of the claimed invention.

The schematic drawings presented herein are not necessarily to scale. Like numbers used in the figures refer to like components, steps and the like. However, it will be understood that the use of a number to refer to a component in a given figure is not intended to limit the component in another figure labeled with the same number. In addition, the use of different numbers to refer to components is not intended to indicate that the different numbered components cannot be the same or similar.

### DETAILED DESCRIPTION

In the following detailed description, reference is made to the accompanying drawings that form a part hereof, and in which are shown by way of illustration several embodiments of devices, systems and uses. It is to be understood that other embodiments are contemplated and may be made without departing from the scope of the present invention as defined by the claims. The following detailed description, therefore, is not to be taken in a limiting sense.

All scientific and technical terms used herein have meanings commonly used in the art unless otherwise specified. The definitions provided herein are to facilitate understanding of certain terms used frequently herein and are not meant to limit the scope of the present disclosure.

As used in this specification and the appended claims, the singular forms "a", "an", and "the" encompass embodiments having plural referents, unless the content clearly dictates otherwise.

As used in this specification and the appended claims, the term "or" is generally employed in its sense including "and/or" unless the content clearly dictates otherwise.

As used herein, "have", "having", "include", "including", "comprise", "comprising" or the like are used in their open ended sense, and generally mean "including, but not limited to."

As used herein, a "patient for which a blood fluid removal session is indicated" is a patient that has undergone, is undergoing, or is likely to undergo at least one blood fluid removal session. In general, such patients are fluid overloaded patients, such as patients suffering from heart failure, chronic kidney disease, or acute kidney failure. Often such patients are stage 3 to stage 5 chronic kidney disease patients, are unresponsive or under-responsive to diuretics, or the like.

As used herein, a "blood fluid removal process," or the like, refers to a process from which fluid is removed from blood of a patient and the blood is returned to the patient. In most cases, the blood is also cleaned; i.e., waste products are removed from the blood and cleaned blood is returned to the patient. Examples of blood fluid removal processes include ultrafiltration, hemofiltration, hemodialysis, hemodiafiltration, peritoneal dialysis, and the like. Any patient for which blood fluid removal is indicated may benefit from the devices, systems and uses described herein.

This disclosure relates to, among other things, systems and uses for monitoring indicators of pH or electrolyte concentrations in patients for which a blood fluid removal process is indicated. In embodiments, the sensors are configured and positioned to monitor pH or electrolytes in one or more of (i) blood of the patient before the blood enters a fluid removal or cleaning medium of the blood fluid removal device; (ii) blood of the patient before after blood exits the medium before being returned to the patient; (iii) fluid removed from the blood of the patient after passing through the medium; (iv) fluid, such as dialysate, before entering the medium; (v) fluid upstream or downstream of the addition of a concentrate for use in altering the composition of the fluid (e.g., dialysate or replacement fluid); (vi) or the like. Additional discussion with regard to sensor placement and use of such sensors will follow. First, however, a brief discussion of blood fluid removal devices or systems that may be used in accordance with the teachings presented herein is presented.

Any suitable device or system for removing fluid, or fluid and contaminants, from blood may be used in accordance with the teachings presented herein. The devices, or components thereof, may be traditional large counsel-type, wearable, or implantable.

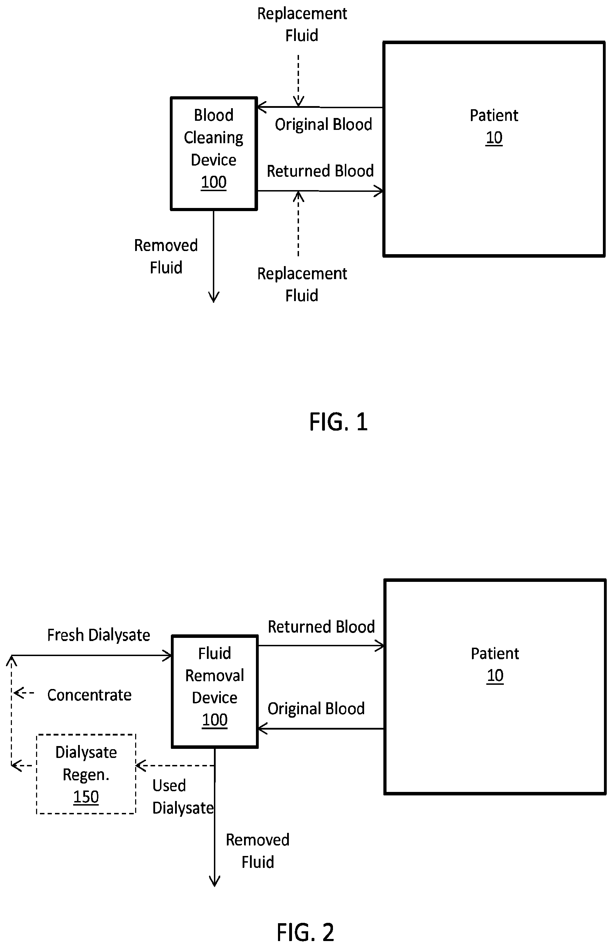

Block diagrams of some example devices and systems are shown in **FIGS. 1-3****.** As shown in **FIG. 1****,** blood may be removed from a patient **10** and fluid may be removed via a blood fluid removal device **100** and returned to the patient **10.** Removed fluid may be diverted. In some embodiments where the blood fluid removal device **100** or system, or components thereof, are implanted, the removed fluid may be diverted to the patient's bladder. Examples of blood fluid removal devices **100** that may operate as depicted in **FIG. 1** are ultrafiltration and hemofiltration devices. Examples of such devices and components thereof that may be employed in accordance with the teachings presented herein are well known in the art. It will be understood that peritoneal dialysis, where dialysate is introduced into the peritoneal cavity, may also be employed.

With some of such devices, replacement fluid may be introduced into the patient's blood if fluid is removed from the blood by the device **100** at too great a rate or amount. The replacement fluid may be added to the original blood before fluid removal or may be added to the blood after initial fluid removal and prior to return to the patient's cardiovascular system. Preferably, the replacement fluid is added after initial fluid removal. The pH and electrolyte concentration of the replacement fluid may be set or adjusted, e.g. as described in more detail below, based on monitoring of pH or electrolytes of the patient.

As shown in the embodiment depicted in **FIG. 2****,** the blood fluid removal device **100** may employ dialysate to assist in removal of contaminants from the patient's blood and in maintaining proper pH and electrolyte balance. The pH or electrolyte concentration of the dialysate may be set or adjusted, e.g. as described in more detail below, based on monitoring of pH or electrolytes. Used dialysate and fluid removed from the blood may be diverted. In some embodiments, particularly where the blood fluid removal device **100** or system or components thereof are wearable or implantable, the used dialysate and removed fluid, or a portion thereof, may be regenerated (indicated by dashed lined regeneration system **150**) to produce fresh dialysate for re-use in the blood fluid removal process. One system for regeneration of dialysate is the REDY system, such as described in Roberts, M, "The regenerative dialysis (REDY) sorbent system," Nephrology 4:275-278, 1998, which system may be employed or readily modified for use in embodiments described herein. As shown in **FIG. 2****,** a concentrate may be added to the regenerated dialysate to adjust the pH and electrolytes of the regenerated dialysate to an amount suitable for re-use as fresh dialysate.

Regardless of whether the dialysate is regenerated, systems and devices that operate in a manner shown in the embodiment of **FIG. 2** include hemodialysis and hemodiafiltration systems. Examples of such devices and components thereof that may be employed in accordance with the teachings presented herein are well known in the art. It will be understood that peritoneal dialysis, where the dialysate is introduced into peritoneal cavity may also be employed.

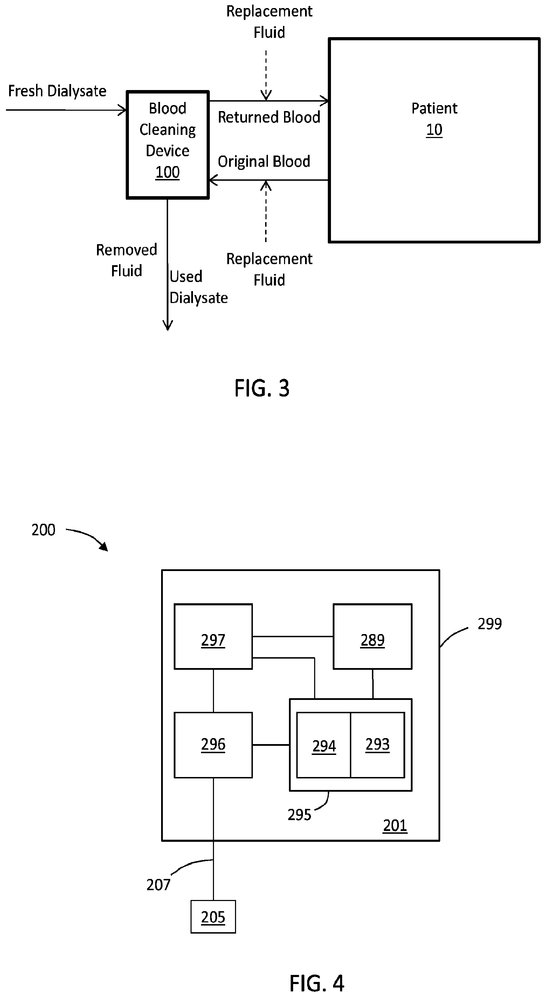

As shown in **FIG. 3****,** in cases where the blood fluid removal device **100** of **FIG. 2** removes fluid from the blood at too high a rate, replacement fluid may be introduced into the patient's blood, upstream or downstream of fluid removal, e.g. as described above with regard to **FIG. 1****.**

Regardless of the device or blood fluid removal process employed, it is important to ensure that the blood pH and electrolyte concentrations are within suitable ranges. If blood electrolyte concentrations are not within suitable ranges, problems with cardiac contractility, efficiency and the like may occur. If the pH is not within a suitable range, acidosis may result, which can result in disruption of cell membranes and denaturation of proteins. In either case, if ranges of blood electrolytes and pH are not properly controlled, the patient's health may be at risk. For example, sudden and cardiac death (including death from congestive heart failure, myocardial infarction, and sudden death) are common in hemodialysis patients. See Bleyer et al, "Sudden and cardiac death rated in hemodialysis patients," Kidney International, (1999), 55:1552-1559.

Accordingly, one goal of hemodialysis, ultrafiltration, and the like is to ensure that the patient's blood pH and electrolyte concentrations are within acceptable ranges. Typical ranges of pH and blood electrolyte concentration that are desired during or following a blood fluid removal session are provided in **Table 1** below. As indicated in **Table 1,** concentrations of various acids or bases (or salts or hydrates thereof) are often important in determining the pH of blood. Accordingly, some typical target concentrations of such acids, bases are presented in **Table 1.**

**Table 1: Typical target ranges for pH and electrolytes (ref. Medical Surgical Nursing, 7^{th} Ed., 2007)**

| | **Target Range** |
|---|---|
| pH | 7.35 - 7.45 |
| Phosphate | 2.8 - 4.5 mg/dL |
| Bicarbonate | 22 - 26 mEq/L |
| Cl⁻ | 96 - 106 mEq/L |
| Mg²⁺ | 1.5 - 2.5 mEq/L |
| Na⁺ | 135 - 145 mEq/L |
| K⁺ | 3.5 - 5.0 mEq/L |
| Ca²⁺ | 4.5 - 5.5 mEq/L |

However, it will be understood that the target for a particular patient may be different from the values presented in **Table 1** for one or more electrolyte or pH. It will also be understood that buffers are typically employed to maintain proper blood pH.

Some suitable buffers that may be used in fluid, such as replacement fluid or dialysate, include bicarbonate, acetate, citrate, lactate, amino acid and protein buffers. The concentration and composition of the buffers and components thereof may be adjusted based on monitored pH of the patient's blood. Similarly, the concentration of electrolytes such as sodium, potassium, calcium, and chloride in replacement fluid or dialysate may be set or altered based the monitored levels of electrolytes.

Any suitable sensor may be employed to monitor pH or electrolytes. For example and referring to **FIG. 4****,** a block diagram showing some components that a sensing device **200** may include is depicted. The sensing device **200** is shown as a stand-alone device in **FIG. 4****,** but it will be understood that the device, one or more components thereof, may be incorporated into other devices, such as a blood fluid cleaning device. The sensor **200** depicted in **FIG. 4** has a housing **299** (which can be shared with another device if the sensor, or portion thereof, is incorporated into the other device) for containing various electronic components **296, 297, 289, 295.** Sensing circuitry **296,** such as analog-to-digital convertor, band-pass filter, or the like, is operably coupled to power supply 297(which, again may be shared) and control electronics **295** (which may be shared), which include a processor **294** and a memory **293** for storing sensed data and processor instructions. Sensing circuitry **296** is also operably coupled to transducer **205,** such as an ion selective electrode, via lead 205. In some embodiments (not shown), the device **200** is leadless, and the transducer **207** or ion selective electrode is exposed through housing **299.** Control electronics **295** are operably coupled to power supply **297** and to communication circuitry **289** for communicating with another device external. In cases where the sensor **200** is a stand-alone device, communication circuitry may be used to communicate with a blood fluid removal device or a device in communication with a blood fluid removal device to transmit data acquired from the sensor to the blood fluid removal device.

Any suitable transducer may be employed to detect pH or electrolytes. In embodiments, the transducer is an ion selective electrode configured to detect H⁺ ions, K⁺ ions, Na⁺ ions, Ca²⁺ ions, Cl⁻ ions, phosphate ions, magnesium ions, acetate ions, amino acids ions, or the like. Such electrodes, and components of sensors employing such electrodes, are known in the art and may be employed, or modified to be employed, for use in the monitoring described herein. One example of a pH sensor is Medtronic, Inc.'s Bravo^{®} pH sensor. One example of a potassium selective electrode is the Thermo Scientific Potassium Ionplus^{®}. Other examples of ion-selective electrodes are contained in the CCX Stat Profile^{®} produced by Nova Biomedical.

In some embodiments, one or more sensors are employed to detect one or more ions to gauge pH or electrolytes in the blood. In some embodiments, a sensor may have more than one transducer, even if leadless, that may monitor more than one ionic species. By measuring more than one ionic species, a more detailed understanding of the levels of various electrolytes or blood components may be had. For example, in some patients in some situations, one electrolyte may be at elevated levels while another may be at reduced levels. In some embodiments, more than one sensor for the same ion is employed for purposes of result confirmation and redundancy, which can improve reliability and accuracy. In some embodiments, sensors for the same ion may be configured to accurately detect different ranges of concentrations of the ion. In embodiments, more than one transducer is present in a single unit. This allows for convenient data collection and circuitry, as all the data may be collected in one place at the same time. Further, the multiple transducers may share the same fluid collection mechanism (e.g., a microdialyzer in the case of an implant), and if needed or desired, may share the same data processing and memory storage components.

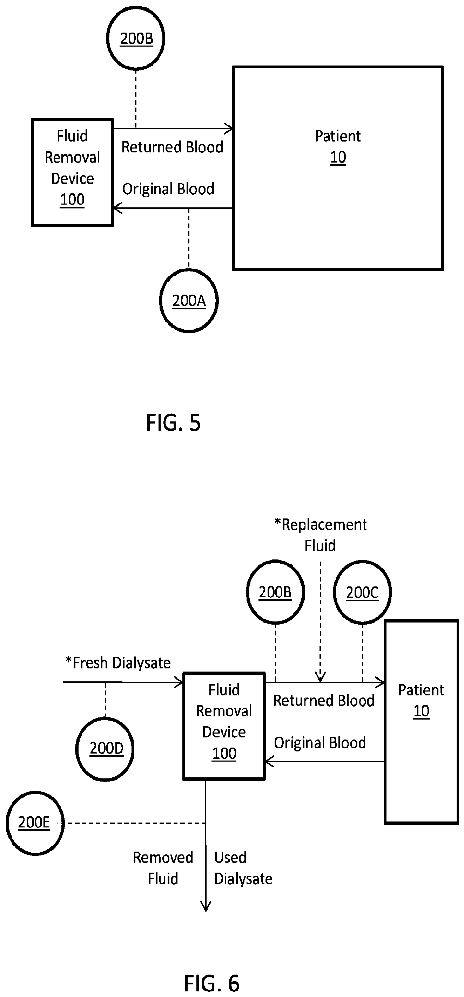

A sensor (or transducer) may be placed at any suitable location for purposes of monitoring electrolytes or pH. For example, and with reference to **FIG. 5****,** an example of a blood fluid removal system and sensors is depicted. One or more of sensors **200A, 200B** may be employed. As shown in **FIG. 5****,** a pH or electrolyte sensor **200A** may be located external to the patient **10** and configured to monitor pH or electrolyte levels in the blood before the blood enters the blood fluid removal device **100** (or before entering blood fluid removal medium, as will be discussed below in more detail). For example, sensor **200A** may be positioned such that a transducer is placed within a catheter carrying blood from the patient **10** to the blood fluid removal device **100** or blood fluid removal media.

Data acquired from a sensor **200A** upstream of the fluid delivery device **100** or blood fluid removal media provides an indication of the actual status of the patient **10.** As a blood fluid cleaning session progresses, data acquired from a sensor **200A** upstream of the fluid delivery device **100** or medium can be used to determine whether blood pH and electrolytes are approaching target ranges or to determine the rate at which pH and electrolytes are changing in the patient as a result of the blood fluid removal process. While not intending to be bound by theory, it is possible that too rapid of a change in pH or electrolyte concentrations can lead to patient hypotension or sudden death that is seen in patient populations that undergo blood fluid removal processes. By monitoring and controlling the rate of change of pH or electrolyte changes in the blood of a patient during the blood fluid removal session, perhaps the incidence of crashing or sudden death can be reduced.

In some embodiments, a sensor **200B** is located external to the patient 10 and configured to monitor pH or electrolyte levels in the blood after the blood exits the blood fluid removal device **100** (or after exiting the blood fluid removal medium) and before being returned to the patient **10.** For example, sensor **200B** may be positioned such that a transducer is placed within a catheter carrying blood from the blood fluid removal device **100** (or medium) to the patient **10.** Such a downstream sensor **200B** may be used to ensure that pH and electrolyte levels of blood to be returned to the patient are not out of range. If the detected levels are out of range or are tending towards out of range, adjustments to pH and electrolyte concentrations can be made to fluid (dialysate or replacement fluid) to avoid introducing fluid into the patient **10** that may cause or exacerbate a cardiac problem associated with electrolyte or pH levels that are too high or too low.

In some embodiments, the system employs both an upstream sensor **200A** and a downstream sensor **200B.** With such systems, the pH or electrolyte levels detected upstream and downstream may be compared, and the compared data may be used to adjust the pH or electrolyte concentration or composition of fluid employed during a blood fluid removal session. The compared data may also be used to determine the rate of change of blood electrolyte concentration or pH. By way of example, prior to a blood fluid removal session or in the early parts of such a session, the patient is typically fluid over-loaded and the concentration of electrolytes may be low (due to the increased fluid volume). It may be appropriate to allow a slightly higher than target concentration electrolyte to be introduced back into the patient when the patient's electrolyte levels are low. However, as the patient's electrolyte levels (as measured by upstream sensor **200A**) approach target levels, the electrolyte levels in the returned blood (as measured by downstream sensor **200B**) should within target range. Monitoring both upstream and downstream will allow for adjustments and checks on progress that may not be attainable by monitoring only one or the other.

Referring now to **FIG. 6****,** a system employing sensors 200B, 200C, 200D, 200E in alternative or additional locations is shown. Any one or more of such sensors 200B, 200C, 200D, 200E may be employed. The system may also employ one or more downstream sensors as described with regard to **FIG. 5****,** but are not shown in **FIG. 6****.** A brief discussion of some possible configurations and uses of sensors 200B, 200C, 200D, 200E is provided herein. However, it will be understood that meaningful data may be obtained from configurations other than those described below.

In embodiments, the system (e.g., the system depicted in **FIG. 6**) employs two upstream sensors 200B, 200C. The first upstream sensor 200B is positioned to monitor pH or electrolyte levels in the blood after it exits the blood fluid removal device **100** or medium but before replacement fluid is added. The second sensor 200C is positioned to monitor pH or electrolyte levels of the blood after the replacement fluid is added and before the blood is returned to the patient **10.** The first sensor 200B may be used to determine what adjustments may be needed to pH and electrolyte levels, and the second sensor 200C may be used to verify that the appropriate adjustments were made to achieve the desired pH and electrolyte concentrations prior to returning the blood to the patient.

In embodiments, the system employs a sensor **200E** to monitor pH or electrolytes removed from the blood of the patient after exiting the blood fluid removal device **100** or medium, and may include a sensor **200D** configured and positioned to monitor pH or electrolytes of fluid (in the depicted case, dialysate) prior to entering the blood fluid device **100** or medium. By monitoring the pH or electrolytes in the fluid as it leaves the device **100** or medium (or the differential pH or electrolyte levels from before entering the device or medium and after exiting the device or medium), the pH or levels of electrolytes (or change in pH or electrolytes) exiting the device **100** or medium may be used to predict the blood pH and electrolyte levels without having to measure the levels in the blood directly. In cases where pH or electrolytes are detected in fluid other than blood and used to derive or predict pH or electrolyte levels or changes in blood, such detection serves as an "indicator" of blood pH or electrolytes. Of course, direct detection in blood also serves as an indicator of blood pH or electrolytes.

Regardless of which sensors **200A-F** (see, **FIGS. 4-6**) are employed, data acquired from the sensors may be used to adjust the pH or electrolyte concentrations of fluid (e.g. dialysate or replacement fluid) used during the dialysis session. In some embodiments, the concentrations of dialysate or replacement fluid are varied and the patient's response to the varying concentrations, as measured by one or more of sensors, is used to determine how best to proceed with further adjustments. In essence, the system may be configured to learn what works best for the particular patient 10. For example, dialysate or replacement fluid having different buffer concentrations or compositions or different electrolyte concentrations may be used during an initial blood fluid session or early in a blood fluid session. The patient's response to these different fluids can be monitored via sensors, and the system can learn what works best for the patient. For example, the system can determine whether the use of the different fluids resulted in the patient's blood levels approaching target levels or deviating from target levels, as well as the rate at which the levels approach or deviate from target ranges. Based on the initial sessions or stages, the system may begin to predict how to react to a particular monitored pH or electrolyte level for the patient and adjust the fluid pH and electrolyte concentrations accordingly.

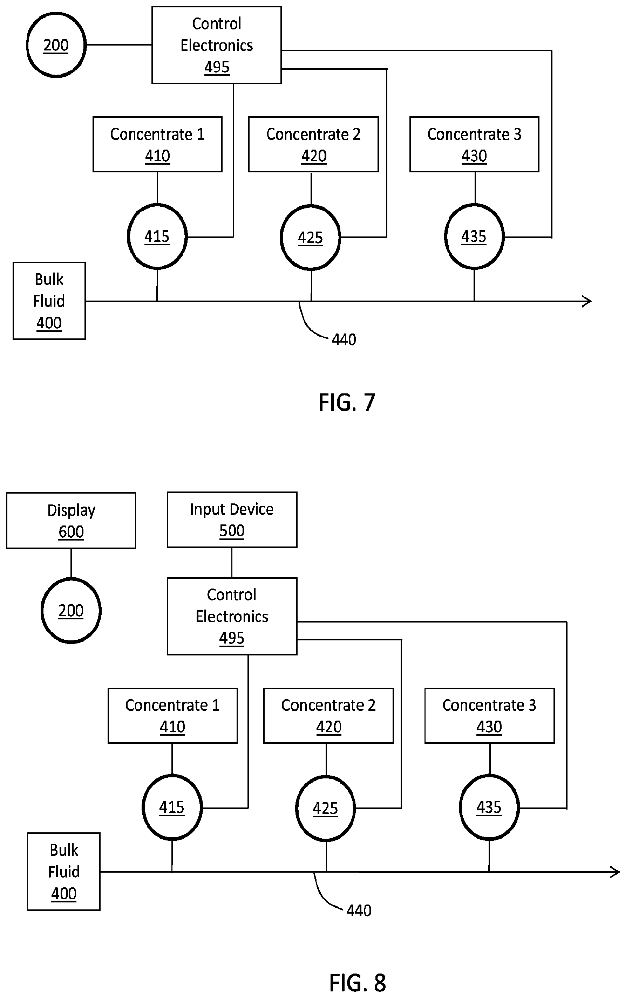

The pH and electrolyte concentration of the fluid (dialysate or replacement fluid) may be adjusted in any suitable manner. For example and with reference to **FIGS. 7-8****,** some representative components of an example of a closed-loop system (**FIG.7**) and an open-loop system **(****FIG. 8****)** for adjusting pH and electrolyte concentrations of fluid are shown. With reference to **FIG. 7****,** data from one or more sensor **200** is presented to control electronics **495,** which are configured to control flow control elements **415, 425, 435,** such as valves. The electronically controllable flow control elements **415, 425, 435** are in fluid communication with supplies of concentrated electrolyte or buffer solutions **410, 420, 430** and with fluid line **440,** which may be a catheter for carrying fresh dialysate or a catheter for carrying replacement fluid. The electronically controllable flow control elements **415, 425, 435,** via control electronics **495,** control the rate at which the concentrates **410, 420, 430** flow into the fluid line **440.** The concentrates **410, 420, 430** are added to bulk fluid **400** to adjust the concentration of electrolytes or the pH of the bulk fluid (and thus the blood).

Referring now to **FIG. 8****,** data from one or more sensor **200** may be processed and appropriate information presented on a display **600,** which may be a part of the blood fluid removal device, a separate computer, or the like. A healthcare provider may use the information presented on the display **600** to adjust the concentration of electrolytes or pH. This can be done, for example, by transmitting appropriate instructions to the control electronics via an input device **500.** Any suitable input device **500** may be used. For example, input device **500** may be a keyboard, a computer, a tablet, a personal data assistant, a physician programmer, or the like. In some embodiments, the input device **500** is the display **600;** e.g., where the display **600** is a touch screen device. Regardless of how the instructions are input, the control electronics **495** can control flow control elements **415, 425, 435** to control the amount of concentrate **410, 420, 430** introduced to bulk fluid **400,** which may be dialysate or replacement fluid.

Any number of suitable concentrates may be used. For example, one concentrate may be sufficient with higher amounts being added when the electrolytes are determined to be low in the patient's blood, and smaller amounts being added when the electrolytes are determined to be high in the patient's blood. More than one concentrate may be used when it is desired to, for example, control pH and electrolyte concentration independently or to control concentration of different electrolytes independently. In embodiments, the number of concentrates is the same as the number of ion species (pH and electrolytes) monitored.

Control elements **415, 425, 435,** as depicted in **FIGS. 7-8** and discussed above, may be any suitable control element, such as electronically controllable valves, electronically controllable pump mechanisms, or the like.

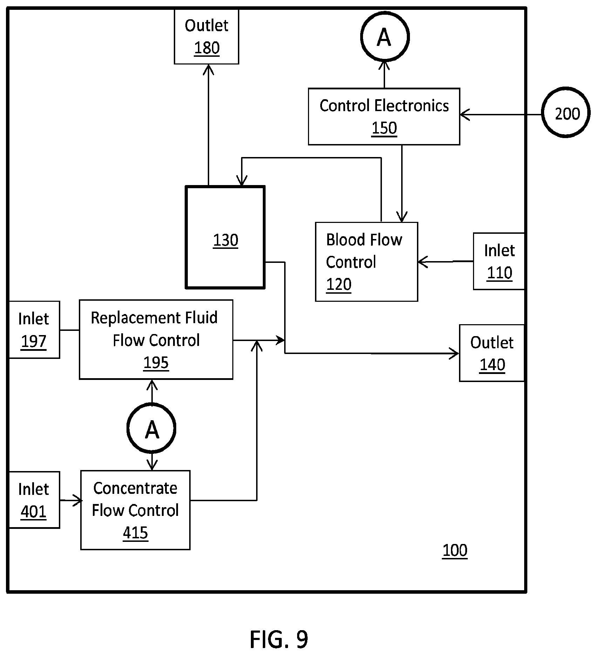

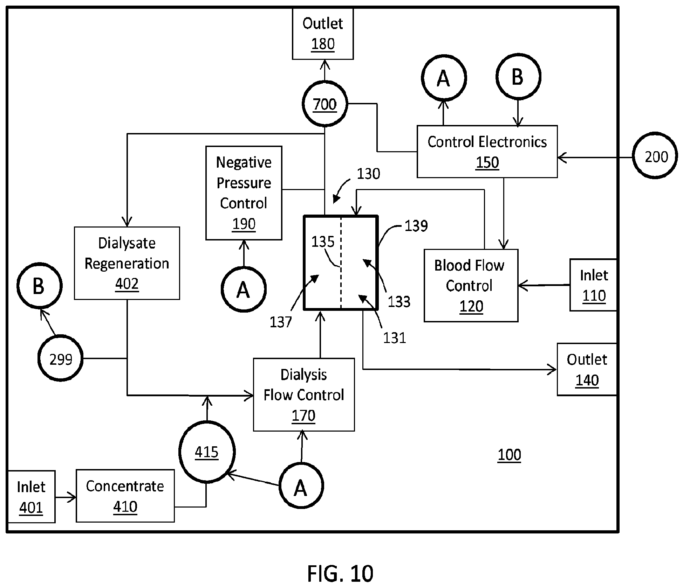

Any suitable system may be configured as depicted in **FIGS. 7-8** to provide control of adjustment of pH or electrolytes based on data acquired from one or more sensors. By way of example, selected components of two example systems are illustrated in **FIGS. 9-****10.** The system in **FIG. 9** illustrates control of flow of a concentrate into replacement fluid, and the system in **FIG. 10** illustrates control of flow of a concentrate into dialysate.

Referring now to **FIG. 9****,** the depicted device **100** includes a fluid pathway for adding replacement fluid to blood before it is returned to the patient. The device **100** includes an inlet **110** for receiving blood from the patient and an outlet **140** for returning blood to the patient. In the flow path between the inlet **110** and outlet **140** are a blood flow control element **120** and a medium for removing fluid and contaminants from the blood. The blood flow control element **120** is operably coupled to control electronics **150** which provide instructions to control the rate at which blood is passed through medium **130.** Fluids and contaminants removed from the blood by the medium **130** may exit via outlet **180.**

The device **100** depicted in **FIG. 9** also includes an inlet **197** for receiving bulk replacement fluid and a replacement fluid flow control element **195** in communication with the inlet and configured to control the rate at which the replacement fluid is added to the blood. The control electronics **150** are operably coupled to the replacement fluid flow control element **195** and are configured to control the rate at which replacement fluid flow control element **195** adds fluid to the blood. The device **100** also includes (i) an inlet **401** for receiving a concentrate for adjusting the pH or electrolyte concentration of the bulk replacement fluid, and (ii) a concentrate flow control element **415** in communication with the inlet **401** and configured to control the rate at which the concentrate is added to the replacement fluid or blood before the blood is returned to the patient. Preferably, the concentrate is added to the replacement fluid prior to the replacement fluid being added to the blood (as depicted) so that the concentrate may be mixed or diluted prior to being added to the blood. The device may include a mixer (not shown) to mix the concentrate and bulk replacement fluid prior to adding to the blood.

In the device depicted in **FIG. 9****,** the control electronics **150** are operably coupled to the concentrate flow control element **415** and are configured to control the rate at which the concentrate flow control element **415** adds fluid to the replacement fluid or blood based on data received from one or more sensors **200** that monitor pH or electrolytes levels (e.g., as described above). By controlling the rate at which the concentrate is introduced into replacement fluid or blood, the concentration or pH (or buffering capacity) of the returned blood can be controlled.

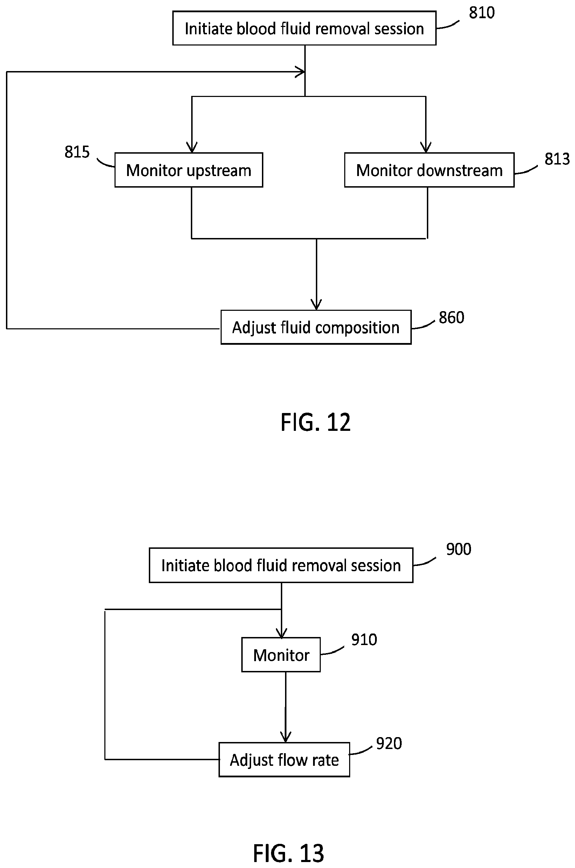

Referring now to **FIG. 10****,** in which components that are numbered the same as in **FIG. 12** refer to the same or similar components, a schematic block diagram of selected components of a blood fluid removal device **100** is shown. In the embodiment depicted in **FIG. 13****,** the device has an inlet **110** for receiving blood from a patient, a blood flow control element **120** in communication with the inlet **110** and configured to control the rate at which blood flows through medium **130** for removing fluid and contaminates from the blood. The device also includes an outlet **140** in communication with the medium **130** for returning blood to the patient. In the depicted embodiment, the medium **130** component has a housing **139** defining a major chamber **131.** A semipermeable filter **135,** such as a hemodialysis or hemodiafiltration membrane filter, sealingly divides the major chamber into two minor chambers **133, 137;** one **133** for blood flow and the other **137** for dialysate flow (as well as fluid and waste that passes through the filter **135** from the blood)

In the embodiment depicted in **FIG. 10****,** used dialysate is regenerated by passing through dialysate regeneration medium **402** or components, such REDY regeneration medium and components, or the like, to regenerate bulk dialysate. The device also has an outlet **180** in communication with the medium **130** for diverting fluid removed from the blood out of the device. A flow regulator element **700,** such as a valve, is operably coupled to control electronics **150** and is disposed in the flow path between the medium **130** and the outlet **180** to control the amount of fluid that exits the device (as a portion of the fluid is regenerated). Often, the regeneration media or components **(402)** remove much of the pH buffer or electrolytes from the dialysate. Accordingly, a concentrate containing concentrated electrolytes and pH buffers is added to the regenerated dialysate before the dialysate re-enters the medium **130.** In some embodiments, a sensor **299** is positioned downstream of the regeneration medium **402** to monitor a level of a component of the regenerated dialysate. The sensor **299** may be a pH or electrolyte sensor and data acquired from sensor **299** may be used in determining how much concentrate to add to the regenerated fluid (which data may be provided to control electronics **150**)**.** The sensor **299** may be a sensor that monitors a blood waste product, such as urea, to determine whether the regeneration media **402** is properly functioning. Increased or detectable levels of a waste product may indicate that the regeneration media **402** or components may need replacement or regeneration.

In the depicted embodiment, the concentrate **410** is stored in a reservoir **410,** having an inlet **401** that allows the concentrate supply in the reservoir **410** to be replenished from time to time. The rate at which the concentrate is added to the regenerated dialysate is controlled by concentrate flow control element **415,** which is operably coupled to control electronics **150,** and is based on data received from sensor **200** that monitors pH or electrolyte concentrations (e.g., as described above).

The device **100** in **FIG. 10** also has a dialysis flow control element **170** for controlling the rate at which dialysis is introduced into the dialysis flow compartment of the medium **130.**

In the depicted embodiment, the device **100** also includes a negative pressure control element **190** in communication with the dialysate compartment of the medium component **130.** The negative pressure control element **190,** which may include a pump or the like, may be used to generate or change a pressure differential across the membrane to control the rate at which fluid is removed from blood that passes though the medium component **130.**

The control electronics **150,** which may include a processor, memory, etc., are operably coupled to, and configured to control, the blood flow control element **120,** the dialysis flow control element **170,** and the negative pressure control element **190.** By controlling these elements in a coordinated manner, the rate at which fluid is removed from blood may be controlled. It will be understood that a device **100** need not have all of the controllable elements **(120, 170, 190)** depicted in **FIG. 10** to effectively control rate of fluid removal from blood.

Any suitable control element may be used for the various control elements **(120, 150, 170, 195, 415)** depicted in **FIGS. 9-10**. For example, a variable or adjustable rate pump may be employed. Alternatively or in addition, a series of electronically controllable valves may be employed. In some embodiments, the valves are in communication flow paths having differing flow resistances.

While **FIGS. 9-10** show devices that can adjust blood electrolyte or pH by adjusting the pH or electrolyte concentration of replacement fluid or dialysate, it will be understood that pH and concentration can also be adjusted by, for example, adjusting the rate at which dialysate or blood is passed over a dialysis membrane. The rate of transfer between blood and dialysate of electrolytes, etc. across the membrane will be dependent on the flow rate of the blood and the dialysate. Accordingly, in systems where dialysate electrolyte concentration or pH cannot be readily adjusted, the rate of flow of blood or dialysate flow may be altered to achieve similar effects to adjusting the concentration of electrolytes in dialysate.

While **FIGS. 9-10** depict components as being within a single unit, it will be understood that one or more of the components may be housed in separate units. For example, the control electronics, or a portion thereof, may be housed in a separate device, such as a computer, tablet, physician programmer, or the like. The computer, tablet, etc. may receive input from sensors, determine appropriate action to take, and instruct appropriate components of a blood fluid removal device to take the appropriate action.

It will be understood that the blood fluid removal devices and systems, and components thereof, described herein are presented for purposes of illustration and not limitation. Components, devices and systems other than those described herein, or derivations of the components, devices and systems described herein, may be employed. Further, components of the devices depicted and discussed above may be interchanged, substituted or added to components of alternative embodiments, as appropriate. Further, it will be understood that, while many of the blood fluid removal devices depicted in a variety of the figures, such as **FIGS. 1-3** and **5-6,** are shown as external to the patient, the teachings presented herein apply if the device, or components thereof, were implanted in the patient.

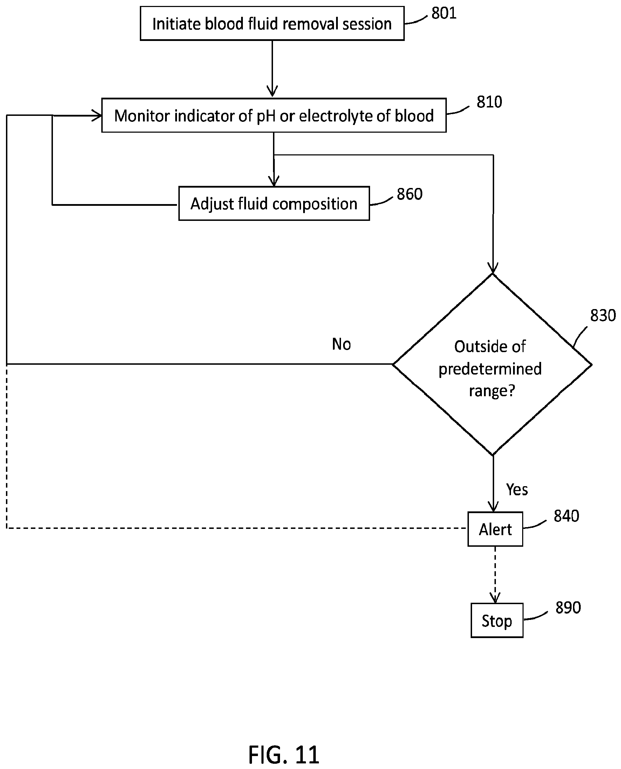

The devices and systems described above, or components thereof, may be used to carry out the uses depicted in **FIGS. 11-13** and described below, or portions thereof. Of course, any suitable device or system may be employed to carry out the uses, or portions thereof, described below. It will be understood that various steps of the uses presented with regard to any one of **FIGS. 11-13** below may be interchanged, substituted, or added to steps presented with regard to any other of **FIGS. 11-13****.**

Referring now to **FIG. 11****,** the depicted use includes initiating a blood fluid removal session **(801)** and monitoring an indicator pH or electrolyte concentration of blood **(810);** e.g. detecting pH or electrolytes in blood or in fluid from which pH or electrolyte levels in blood can be derived. Based on the monitored indicator of pH or electrolytes, the pH or electrolyte composition or concentration of fluid (e.g., dialysate or replacement fluid) used in the blood fluid removal session may be adjusted **(860).** For example, based on one or more of the current value of a monitored ionic species or the rate of change in the monitored ionic species, the fluid composition may be adjusted, e.g. as discussed above.

As shown in **FIG. 11****,** continuous, periodic or intermittent determinations may be made as to whether the pH or electrolyte concentration is out of range **(830)** based on data acquired during the monitoring **(810).** For example, a determination **(830)** may be made as to whether pH or electrolyte levels crossed a threshold (e.g., a ceiling or floor). If the pH or electrolytes are determined to be within range, monitoring **(810)** may continue. If the pH or electrolytes are determined to be out of range (e.g., cross a threshold), an alert **(840)** may be issued to notify the patient or a healthcare provider of the situation. In some cases, the situation may warrant stopping **(890)** of the blood fluid removal session; e.g., if the detected pH or electrolytes are too far out of range or cross a heightened threshold. In other cases, it may be suitable to continue with the blood fluid removal session with heightened awareness of a situation for which increased attention may be warranted.

Referring now to **FIG. 12****,** the depicted use includes initiating a blood fluid removal session **(801)** and monitoring an indicator pH or electrolyte concentration upstream **(815)** and downstream **(813)** of blood fluid removal. Data acquired from upstream and downstream sensors may be compared to determine how to adjust **(860)** the fluid composition, e.g. as described above.

Referring now to **FIG. 13****,** the depicted use shows a use where blood electrolyte concentration or pH is adjusted by altering the flow rate of dialysate or blood. The use includes initiating a blood fluid removal session **(900),** such as a hemodialysis session, and monitoring an indicator of pH or electrolyte **(910),** which can be in the patient, upstream of the device, downstream of the device, within the device, or the like. Based on the monitored data **(910),** adjustments to the flow of dialysate or blood may be made **(920)** to adjust the electrolyte concentration or pH in the blood that gets returned to the patient.

The uses described herein, including the uses depicted in **FIGS. 11-13****,** may be carried out by sensor devices, blood fluid removal devices, or other devices in communication with sensor devices or blood fluid removal devices. These uses may be algorithms or instructions programmed into memory of such devices, which may be carried out by processors or other control electronics of the devices. Preferably, the processor is in communication with appropriate control elements of the devices and is configured to control such elements in a manner such that the programmed instructions are carried out by the appropriate device. It will be understood that a computer readable medium programmed with instructions that cause a sensor device, blood fluid removal device, or other suitable device to carry out a use, or a portion thereof, as described herein are contemplated. The computer readable medium may be non-transitory, i.e. lasting for more than a fleeting instant or seconds. The medium may be memory, such as RAM or ROM, a cd or dvd, flash memory, or the like.

A variety of aspects of uses, systems, devices, computer-readable media and the like are disclosed herein.

Thus, systems, devices and uses for ELECTROLYTE AND pH MONITORING FOR FLUID REMOVAL PROCESSES are described. Those skilled in the art will recognize that the preferred embodiments described herein may be altered or amended without departing from the true scope of the invention, as defined by the accompanying claims.

In the claims that follow, the designators "first", "second", "third" and the like are used for purposes of distinguishing between elements and not for purposes of enumerating the elements or for defining a sequence of the elements. For example, a "third" sensor does not necessarily imply that there are three sensors but rather that the "third" sensor is distinct from the "first" sensor. By way of further example, a "third" sensor does not necessarily come later in time than a "first" sensor.

## Claims

1. A system comprising:
a blood fluid removal device (100) comprising
(i) a first inlet (110) for receiving blood from a patient,
(ii) a first outlet for returning blood to the patient,
(iii) a second inlet for receiving fresh dialysate,
(iv) a second outlet for outflow of used dialysate,
(v) a medium (130) for removing fluid and contaminants from the blood, the medium being positioned between the first inlet and the first outlet and between the second inlet and the second outlet,
(vi) a source (400) of bulk replacement fluid, and
(vii) a fluid path (440) carrying the bulk replacement fluid from the source (400) to the blood after the blood exits the medium;
a concentrate source (410, 420, 430) housing a concentrate solution comprising concentrated electrolyte or pH buffer;
a concentrate flow control element (415, 425, 435) configured to control the rate at which the concentrate solution enters the bulk replacement fluid in the fluid path from the concentrate source thereby forming replacement fluid which enters the blood after the blood exits the medium;
a first sensor (200B) configured to monitor a first indicator of blood electrolyte concentration or blood pH in the blood after the blood exits the medium but before the replacement fluid is added;
a second sensor (200C) configured to monitor the first indicator of blood electrolyte concentration or blood pH in the blood after the replacement fluid is added; and
control electronics (495) in operable communication with the first and second sensors and the concentrate flow control element,
wherein the control electronics (495) are configured to compare first indicator data acquired from the first sensor (200B) to first indicator data acquired from the second sensor (200C) to determine a rate of exchange of blood pH or blood electrolyte concentration and wherein the control electronics (495) are configured, via the flow control element, to adjust the rate at which the concentrate solution enters the fluid path based on the rate of change determined from the comparison of data acquired from the first sensor (200B) and the second sensor (200C); and
wherein a third sensor (200E) is configured to monitor a second indicator of electrolyte concentration or pH in used dialysate after the used dialysate exits the medium; and
further comprising a fourth sensor (200D) configured to monitor the second indicator of electrolyte concentration or pH in fresh dialysate in the fluid path before the dialysate enters the medium, wherein the control electronics are in operable communication with the fourth sensor and are configured to compare second indicator data acquired from the third sensor to second indicator data acquired from the fourth sensor, and wherein the control electronics are configured to adjust the rate at which the concentrate solution enters the fluid path (440) based on the comparison of the data acquired from the third sensor and the fourth sensor

2. The system of claim 1, further comprising a computer readable medium, wherein the computer readable medium comprises instructions that cause the control electronics to control the concentrate flow control element to adjust the rate at which the concentrate solution enters the fluid path based on data obtained from the first and second sensors.

3. The system of any preceding claim, wherein said fluid removal device comprises: a medium housing for the medium, and defining a major chamber;
a blood flow removal membrane disposed in the housing and sealingly dividing the major chamber into first and second minor chambers;
a first inlet and a first outlet in fluid communication with the first minor chamber, wherein the system is configured such that blood enters the first minor chamber through the first inlet and exits the first minor chamber through the first outlet;
a second inlet and a second outlet in fluid communication with the second minor chamber, wherein the system is configured such that dialysate enters the second minor chamber through the second inlet and exits the second minor chamber through the second outlet;
the medium being a dialysate regeneration medium in fluid communication with and disposed in a dialysate flow path between the second inlet and the second outlet.

## Patentansprüche

1. System, umfassend:
eine Blutfluidentfernungsvorrichtung (100), umfassend
(i) einen ersten Einlass (110) zum Aufnehmen von Blut von einem Patienten,
(ii) einen ersten Auslass zum Zurückführen von Blut an den Patienten,
(iii) einen zweiten Einlass zum Aufnehmen von frischem Dialysat,
(iv) einen zweiten Auslass für eine Ausströmung von gebrauchtem Dialysat,
(v) ein Medium (130) zum Entfernen von Fluid und Verunreinigungen aus dem Blut, wobei das Medium zwischen dem ersten Einlass und dem ersten Auslass und zwischen dem zweiten Einlass und dem zweiten Auslass positioniert ist,
(vi) eine Quelle (400) eines Hauptmengen-Substitutionsfluids, und
(vii) einen Fluidpfad (440), der das Hauptmengen-Substitutionsfluid von der Quelle (400) zu dem Blut befördert, nachdem das Blut aus dem Medium austritt;
eine Konzentratquelle (410, 420, 430), die eine Konzentratlösung, umfassend einen konzentrierten Elektrolyten oder einen pH-Puffer, umhaust;
ein Konzentratströmungssteuerelement (415, 425, 435), das konfiguriert ist, um die Rate zu steuern, mit der die Konzentratlösung von der Konzentratquelle in das Hauptmengen-Substitutionsfluid in dem Fluidpfad eintritt, wobei dadurch ein Substitutionsfluid ausgebildet wird, das in das Blut eintritt, nachdem das Blut aus dem Medium austritt;
einen ersten Sensor (200B), der konfiguriert ist, um einen ersten Indikator einer Blutelektrolytkonzentration oder eines Blut-pH-Werts in dem Blut zu überwachen, nachdem das Blut aus dem Medium austritt, aber bevor das Substitutionsfluid hinzugefügt wird;
einen zweiten Sensor (200C), der konfiguriert ist, um den ersten Indikator der Blutelektrolytkonzentration oder des Blut-pH-Werts in dem Blut zu überwachen, nachdem das Substitutionsfluid hinzugefügt wird; und
eine Steuerelektronik (495) in Wirkverbindung mit dem ersten und dem zweiten Sensor und dem Konzentratströmungssteuerelement,
wobei die Steuerelektronik (495) konfiguriert ist, um von dem ersten Sensor (200B) erfasste erste Indikatordaten mit von dem zweiten Sensor (200C) erfassten ersten Indikatordaten zu vergleichen, um eine Rate eines Austauschs des Blut-pH-Werts oder der Blutelektrolytkonzentration zu bestimmen, und wobei die Steuerelektronik (495) konfiguriert ist, um über das Strömungssteuerelement die Rate zu justieren, mit der die Konzentratlösung in den Fluidpfad eintritt, basierend auf der Änderungsrate, die aus dem Vergleich von Daten bestimmt wird, die von dem ersten Sensor (200B) und dem zweiten Sensor (200C) erfasst werden; und
wobei ein dritter Sensor (200E) konfiguriert ist, um einen zweiten Indikator der Elektrolytkonzentration oder des pH-Werts in gebrauchtem Dialysat zu überwachen, nachdem das gebrauchte Dialysat aus dem Medium austritt; und
ferner umfassend einen vierten Sensor (200D), der konfiguriert ist, um den zweiten Indikator der Elektrolytkonzentration oder des pH-Werts in frischem Dialysat in dem Fluidpfad zu überwachen, bevor das Dialysat in das Medium eintritt, wobei die Steuerelektronik in Wirkverbindung mit dem vierten Sensor steht und konfiguriert ist, um von dem dritten Sensor erfasste zweite Indikatordaten mit von dem vierten Sensor erfassten zweiten Indikatordaten zu vergleichen, und wobei die Steuerelektronik konfiguriert ist, um die Rate zu justieren, mit der die Konzentratlösung in den Fluidpfad (440) eintritt, basierend auf dem Vergleich der Daten, die von dem dritten Sensor und dem vierten Sensor erfasst werden.

2. System nach Anspruch 1, ferner umfassend ein computerlesbares Medium, wobei das computerlesbare Medium Anweisungen umfasst, die die Steuerelektronik veranlassen, das Konzentratströmungssteuerelement zu steuern, um die Rate zu justieren, mit der die Konzentratlösung in den Fluidpfad eintritt, basierend auf Daten, die von dem ersten und dem zweiten Sensor erhalten werden.

3. System nach einem der vorstehenden Ansprüche, wobei die Fluidentfernungsvorrichtung umfasst: ein Mediumgehäuse für das Medium, und das eine Hauptkammer definiert;
eine Blutströmungsentfernungsmembran, die in dem Gehäuse angeordnet ist und die Hauptkammer in eine erste und eine zweite Nebenkammer abdichtend unterteilt;
einen ersten Einlass und einen ersten Auslass in Fluidverbindung mit der ersten Nebenkammer, wobei das System derart konfiguriert ist, dass Blut durch den ersten Einlass in die erste Nebenkammer eintritt und durch den ersten Auslass aus der ersten Nebenkammer austritt;
einen zweiten Einlass und einen zweiten Auslass in Fluidverbindung mit der zweiten Nebenkammer, wobei das System derart konfiguriert ist, dass Dialysat durch den zweiten Einlass in die zweite Nebenkammer eintritt und durch den zweiten Auslass aus der zweiten Nebenkammer austritt;
wobei das Medium ein Dialysatregenerationsmedium ist, das in Fluidverbindung mit einem Dialysatströmungspfad zwischen dem zweiten Einlass und dem zweiten Auslass steht und in diesem angeordnet ist.

## Revendications

1. Système comprenant :
un dispositif de retrait de fluide sanguin (100) comprenant
(i) une première entrée (110) destinée à recevoir du sang d'un patient,
(ii) une première sortie destinée à renvoyer le sang au patient,
(iii) une seconde entrée destinée à recevoir du dialysat frais,
(iv) une seconde sortie destinée à l'écoulement sortant du dialysat usé,
(v) un milieu (130) destiné à retirer du fluide et des contaminants du sang, le milieu étant positionné entre la première entrée et la première sortie et entre la seconde entrée et la seconde sortie,
(vi) une source (400) de fluide de substitution majoritaire, et
(vii) un trajet de fluide (440) acheminant le fluide de substitution majoritaire depuis la source (400) vers le sang après que la sortie du sang du milieu ;
une source de concentré (410, 420, 430) logeant une solution concentrée comprenant un électrolyte concentré ou un tampon de pH ;
un élément de contrôle d'écoulement de concentré (415, 425, 435) configuré pour contrôler le débit auquel la solution concentrée pénètre dans le fluide de substitution majoritaire dans le trajet de fluide depuis la source de concentré formant de ce fait un fluide de substitution qui pénètre dans le sang après la sortie du sang du milieu ;
un premier capteur (200B) configuré pour surveiller un premier indicateur de concentration d'électrolytes sanguins ou de pH sanguin dans le sang après la sortie du sang du milieu, mais avant que le fluide de substitution ne soit ajouté ;
un deuxième capteur (200C) configuré pour surveiller le premier indicateur de concentration d'électrolytes sanguins ou de pH sanguin dans le sang après que le fluide de substitution a été ajouté ; et
une électronique de contrôle (495) en communication fonctionnelle avec les premier et deuxième capteurs et l'élément de contrôle d'écoulement de concentré,
dans lequel l'électronique de contrôle (495) est configurée pour comparer des premières données d'indicateur acquises à partir du premier capteur (200B) à des premières données d'indicateur acquises à partir du deuxième capteur (200C) pour déterminer un débit d'échange de pH sanguin ou de concentration d'électrolytes sanguins, et dans lequel l'électronique de contrôle (495) est configurée pour ajuster, par l'intermédiaire de l'élément de contrôle d'écoulement, le débit auquel la solution concentrée pénètre dans le trajet de fluide en fonction du débit de variation déterminé à partir de la comparaison de données acquises à partir du premier capteur (200B) et du deuxième capteur (200C) ; et
dans lequel un troisième capteur (200E) est configuré pour surveiller un second indicateur de concentration d'électrolytes ou de pH dans le dialysat usé après que le dialysat usé est sorti du milieu ; et
comprenant en outre un quatrième capteur (200D) configuré pour surveiller le second indicateur de concentration d'électrolytes ou de pH dans le dialysat frais dans le trajet de fluide avant que le dialysat ne pénètre dans le milieu, dans lequel l'électronique de contrôle est en communication fonctionnelle avec le quatrième capteur et est configurée pour comparer des données du second indicateur acquises à partir du troisième capteur à des données du second indicateur acquises à partir du quatrième capteur, et dans lequel l'électronique de contrôle est configurée pour ajuster le débit auquel la solution concentrée pénètre dans le trajet de fluide (440) en fonction de la comparaison des données acquises à partir du troisième capteur et du quatrième capteur

2. Système selon la revendication 1, comprenant en outre un support lisible par ordinateur, dans lequel le support lisible par ordinateur comprend des instructions qui amènent l'électronique de contrôle à contrôler l'élément de contrôle d'écoulement de concentré pour ajuster le débit auquel la solution concentrée pénètre dans le trajet de fluide en fonction de données obtenues à partir des premier et deuxième capteurs.

3. Système selon l'une quelconque revendication précédente, dans lequel ledit dispositif de retrait de fluide comprend : un logement de milieu pour le milieu, et définissant une chambre principale ;
une membrane de retrait d'écoulement sanguin disposée dans le logement et divisant de manière étanche la chambre principale en des première et seconde chambres secondaires ;
une première entrée et une première sortie en communication de fluide avec la première chambre mineure, dans lequel le système est configuré de telle sorte que le sang pénètre dans la première chambre mineure à travers la première entrée et sort de la première chambre mineure à travers la première sortie ;
une seconde entrée et une seconde sortie en communication de fluide avec la seconde chambre mineure, dans lequel le système est configuré de telle sorte que le dialysat pénètre dans la seconde chambre mineure à travers la seconde entrée et sort de la seconde chambre mineure à travers la seconde sortie ;
le milieu étant un milieu de régénération de dialysat en communication de fluide avec et disposé dans un trajet d'écoulement de dialysat entre la seconde entrée et la seconde sortie.
